# EUROPÄISCHE PATENTSCHRIFT

(11) **EP 3 331 461 B1**
(45) Veröffentlichungstag und Bekanntmachung des Hinweises auf die Patenterteilung: **20.04.2022**
(21) Anmeldenummer: 16753300.9
(22) Anmeldetag: 03.08.2016
(51) Int. Cl.: A61B 17/70

(54) **HANDHABUNGSINSTRUMENT FÜR EINEN KNOCHENANKER**
HANDLING INSTRUMENT FOR A BONE ANCHOR
INSTRUMENT DE PRÉHENSION POUR UN ANCRAGE OSSEUX

(30) Priorität: 04.08.2015 DE 102015214874
(43) Veröffentlichungstag der Anmeldung: 13.06.2018
(73) Patentinhaber: Silony Medical International AG, 8500 Frauenfeld (CH)
(72) Erfinder: HEUER, Frank, 70794 Filderstadt (DE); RIESINGER, Ralf, 78234 Engen (DE); OHNMACHT, Timo, 78736 Trichtingen (DE)
(74) Vertreter: DREISS Patentanwälte PartG mbB
(86) Internationale Anmeldenummer: PCT/EP2016/068582
(87) Internationale Veröffentlichungsnummer: WO 2017/021469

(56) Entgegenhaltungen:
- EP-A1- 2 692 304
- US-A1- 2008 319 477
- US-A1- 2012 253 413
- US-A1- 2013 204 263
- US-B1- 8 439 922

## Beschreibung

Die Erfindung betrifft ein Handhabungsinstrument für einen Knochenanker, insbesondere eine Knochenschraube, insbesondere eine Pedikelschraube in der Wirbelsäulenchirurgie, wobei das Instrument mit einem Kopf des Knochenankers lösbar, jedoch starr und drehfest verbindbar ist und in einer axialen Längsrichtung erstreckt ist, mit einem in der Längsrichtung erstreckten außenliegenden ersten Gehäuseteil und mit einem in der Längsrichtung erstreckten in das außenliegende erste Gehäuseteil in der Längsrichtung einführbaren innenliegenden zweiten Gehäuseteil, wobei die Gehäuseteile wenigstens abschnittsweise rohr- oder hülsenförmig ausgebildet sind und das außenliegende erste Gehäuseteil ein in der Längsrichtung erstrecktes Innengewinde und das innenliegende zweite Gehäuseteil ein in der Längsrichtung erstrecktes Außengewinde aufweist, das in das Innengewinde einschraubbar ist, wobei das außenliegende erste Gehäuseteil den Kopf des Knochenankers in der Längsrichtung hintergreift und das innenliegende zweite Gehäuseteil im eingeschraubten Zustand gegen den Kopf des Knochenankers in der Längsrichtung abstützbar ist, so dass das Instrument hierdurch am Kopf des Knochenankers lösbar fixierbar ist.

Derartige Handhabungsinstrumente sind bekannt. Ein Handhabungsinstrument mit den Merkmalen des Oberbegriffs des Anspruchs 1 ist aus der US 8,439,922 B1 bekannt. Ein weiteres Handhabungsinstrument ist aus der US 2013/204263 A1 bekannt.

Demgemäß liegt der vorliegenden Erfindung die Aufgabe zugrunde, das Handhabungsinstrument dahingehend zu verbessern, dass es komfortabler für den Chirurgen anwendbar ist.

Diese Aufgabe wird bei einem Handhabungsinstrument der genannten Art erfindungsgemäße dadurch gelöst, dass das außenliegende erste Gehäuseteil im Bereich des Innengewindes einen gewindelosen Abschnitt aufweist, der von einem proximalen Längsende des Innengewindes ausgehend in der Längsrichtung distal erstreckt ist und über ein Winkelsegment in Umfangsrichtung erstreckt ist, und dass das Außengewinde des innenliegenden zweiten Gehäuseteils ausgehend von einem distalen Längsende des Außengewindes nur im Bereich der Erstreckung des gewindelosen Abschnitts des ersten außenliegenden Gehäuseteils ausgebildet ist, so dass das zweite innenliegende Gehäuseteil bei geeigneter Drehstellung in der Längsrichtung in das erste außenliegende Gehäuseteil translatorisch einsteckbar ist, bis das distale Längsende des Außengewindes auf ein distales Ende des gewindelosen Abschnitts, d.h. auf das Innengewinde, trifft und im Folgenden in das Innengewinde des ersten außenliegenden Gehäuseteils einschraubbar ist. Durch die erfindungsgemäße Ausbildung des Handhabungsinstruments braucht das innenliegende zweite Gehäuseteil beim Fügen mit dem außenliegenden ersten Gehäuseteil nicht über seinen gesamten Stellweg geschraubt zu werden, sondern - wie vorausgehend ausgeführt - kann das innenliegende Gehäuseteil zu Beginn des Fügevorgangs translatorisch in das außenliegende Gehäuseteil eingesteckt werden. Dies wird erfindungsgemäß dadurch ermöglicht, dass bei dem innenliegenden Gehäuseteil das Außengewinde erfindungsgemäß nicht in Umfangsrichtung durchgehend ausgebildet ist, sondern eben nur in dem erwähnten in der Längsrichtung erstreckten Bereich, der sich innerhalb der Erstreckung des gewindelosen Abschnitts bei dem außenliegenden Gehäuseteil befindet. Genau dies ermöglicht die translatorische Steckbewegung zu Beginn des Fügevorgangs. Das innenliegende Gehäuseteil lässt sich also in der Längsrichtung bei geeigneter Drehstellung über die Längserstreckung des gewindelosen Abschnitts translatorisch fügen oder stecken, bis das distale Längsende des Außengewindeabschnitts am distalen Ende des gewindelosen Abschnitts auf das vorzugsweise in der vollen Umfangsrichtung erstreckte Innengewinde des außenliegenden Gehäuseteils trifft. Ab diesem Zeitpunkt wird das innenliegende Gehäuseteil durch eine rotatorische Schraubbewegung in das außenliegende Gehäuseteil eingeschraubt.

Zur Ausbildung des gewindelosen Abschnitts bei dem ersten außenliegenden Gehäuseteil könnte beispielsweise zunächst ein in Umfangsrichtung durchgehendes Innengewinde ausgebildet werden, welches dann bereichsweise wieder, etwa durch spanabhebende Bearbeitung, entfernt wird. Demgegenüber kann es sich als vorteilhaft erweisen, wenn der gewindelose Abschnitt des ersten außenliegenden Gehäuseteils dadurch gebildet ist, dass das erste außenliegende Gehäuseteil dort eine radial zur Längsrichtung ausgebildete und nach außen durchgehende Ausnehmung in seiner Wandung aufweist. Die Ausnehmung lässt sich beispielsweise im noch ebenen Flachmaterialzustand des außenliegenden Gehäuseteils herstellen, etwa durch einen einfachen Stanzvorgang. Ein wesentlicher weiterer Vorteil besteht darin, dass der Anwender anhand der durchgehenden fensterartigen Ausnehmung unmittelbar erkennt, wo der gewindelose Abschnitt im ersten außenliegenden Gehäuseteil ausgebildet ist. Dies gestattet die Positionierung des innenliegenden zweiten Gehäuseteils in der Drehstellung intuitiv und einfach.

Hinsichtlich der Abmessungen des gewindelosen Abschnitts des ersten außenliegenden Gehäuseteils erweist es sich als vorteilhaft, wenn der gewindelose Abschnitt in Umfangsrichtung eine Erstreckung von 3 bis 20 mm und in der Längsrichtung von 5 bis 100 mm aufweist.

Weiter erweist es sich hinsichtlich der Erzielung einer stabilen Verbindung zwischen den Gehäuseteilen als vorteilhaft, wenn das erste außenliegende Gehäuseteil zwei gewindelose Abschnitte aufweist, die diametral zur Längsrichtung einander gegenüberliegend ausgebildet sind. Dies eröffnet die Möglichkeit, dass auch das innenliegende Gehäuseteil auf diametral gegenüberliegenden Seiten je einen Außengewindeabschnitt aufweist. Hierdurch werden die Kräfte beim Verschrauben der beiden Teile gleichmäßiger verteilt und aufgenommen.

Das erste außenliegende Gehäuseteil könnte beispielsweise eine starre im Wesentlichen zylindrische Gestalt aufweisen und über Ausnehmungen und Vorsprünge in eine Hintergriffstellung mit dem Kopf des Knochenankers gebracht werden. Demgegenüber erweist es sich als vorteilhaft, wenn das erste außenliegende Gehäuseteil zwei diametral zueinander angeordnete halbschalenförmige Schenkel aufweist, die derart geringfügig quer zur Längsrichtung gegeneinander auslenkbar sind, dass sie in eine Hintergriffsstellung am Kopf des Knochenankers gelangen, insbesondere einrasten oder einschnappen, können und dabei ein proximales Ende des Kopfs des Knochenankers zwischen sich aufnehmen. Auf diese Weise lässt sich bei einer federnden Auslenkbarkeit der halbschalenförmige Schenkel eine Rückstellkraft zur spielfreien Aufnahme am Kopf des Knochenankers realisieren.

Weiterhin erweist es sich als vorteilhaft, wenn das zweite innenliegende Gehäuseteil einen ersten distalen Abschnitt und einen das Außengewinde aufweisenden proximalen Abschnitt aufweist, die über ein Drehgelenk gegeneinander um die axiale Längsrichtung verdrehbar sind. Auf diese Weise wird bei einem Verschrauben des innenliegenden zweiten Gehäuseteils mit dem außenliegenden ersten Gehäuseteil nur der proximale Abschnitt des innenliegenden Gehäuseteils in Umfangsrichtung gedreht, der distale Abschnitt ist von der Drehbewegung über das Drehgelenk entkoppelt und wird weiterhin translatorisch eingeführt.

Nach einer vorteilhaften Ausgestaltung der Erfindung sind Längsführungsmittel für eine geführte Aufnahme des innenliegenden zweiten Gehäuseteils in das außenliegende erste Gehäuseteil vorgesehen. Über diese ist der erste distale Abschnitt des zweiten innenliegenden Gehäuseteils unverdrehbar zu dem ersten außenliegenden Gehäuseteils darin geführt aufnehmbar.

Weiterhin wäre es denkbar, dass die Längsführungsmittel die Aufnahme des zweiten innenliegenden Gehäuseteils in dem ersten außenliegenden Gehäuseteil nur in einer diskreten Drehstellung zulassen.

Nach einer weiteren vorteilhaften Ausgestaltung der Erfindung weist das erste außenliegende Gehäuseteil in einem distalen Endbereich ein weiteres distales Längsführungsmittel auf. Dieses kann beispielsweise durch mindestens einen nach radial innen vorstehenden Vorsprung, der insbesondere pilzförmig oder T-förmig ausgebildet ist, in dem distalen Endbereich des außenliegenden Gehäuseteils ausgebildet sein. Das zweite innenliegende Gehäuseteil weist eine mit dem Vorsprung zusammenwirkende Ausnehmung auf, die vorteilhafterweise ausgehend von einem von einem distalen Ende des zweiten innenliegenden Gehäuseteils, randoffen und in der Längsrichtung erstreckt ausgebildet ist. Über diese Längsführungsmittel wird bei einem Verschrauben des innenliegenden zweiten Gehäuseteils mit dem außenliegenden ersten Gehäuseteil der distale Abschnitt des innenliegenden zweiten Gehäuseteils, der von Drehbewegung über das Drehgelenk entkoppelt ist, in Längsrichtung geführt.

Nach einer weiteren vorteilhaften Ausgestaltung der Erfindung weist das erste außenliegende Gehäuseteil eine insbesondere radiale Ausnehmung auf, durch welche die Position des zweiten innenliegenden Gehäuseteils in Längsrichtung relativ zu dem ersten außenliegenden Gehäuseteil, visuell wahrnehmbar ist. Dies kann dadurch ermöglicht sein, dass das zweite innenliegende Gehäuseteil eine visuell wahrnehmbare Markierung an der Außenseite aufweist. Dies gestattet die gewünschte korrekte Positionierung des innenliegenden zweiten Gehäuseteils in Längsrichtung relativ zu dem ersten außenliegenden Gehäuseteil.

Nach einer weiteren vorteilhaften Ausgestaltung der Erfindung weist das zweite innenliegende Gehäuseteil an einem proximalen Ende eine oder mehrere Werkzeugansetzstellen, auf um ein komfortableres Verschrauben des innenliegenden zweiten Gehäuseteils in das außenliegende erste Gehäuseteil zu ermöglichen. An diesen Ansetzstellen kann ein passendes Werkzeug so angesetzt werden, dass das innenliegende zweite Gehäuseteil damit um die Längsrichtung gedreht werden kann.

Bevorzugte Weiterbildungen sind in den abhängigen Ansprüchen beschrieben, für die Schutz in Anspruch genommen wird, sowie aus der zeichnerischen Darstellung und nachfolgenden Beschreibung eines bevorzugten Ausführungsbeispiels des erfindungsgemäßen Handhabungsinstruments. In der Zeichnung zeigt:
Figuren 1,2 und 3 eine respektive Darstellung des erfindungsgemäßen Handhabungsinstruments;
Figur 4 eine Einzelheit aus Figur 2;
Figur 5 eine Einzelheit aus Figur 3;
Figur 6 eine Längsschnittansicht des erfindungsgemäßen Handhabungsinstrumentes nach Figur 1 und eines darin befindlichen Stabdruckteils nach Figur 7 und
Figur 7 eine Abbildung des Stabdruckteils.

Die Figuren zeigen ein insgesamt mit dem Bezugszeichen 2 bezeichnetes erfindungsgemäßes Handhabungsinstrument. Es umfasst ein außenliegendes erstes Gehäuseteil 4 und ein innenliegendes zweites Gehäuseteil 6 sowie eine axiale Längsrichtung 8, eine Umfangsrichtung 10 und eine radiale Richtung 12. Die beiden beispielhaft dargestellten Gehäuseteile sind ungefähr rohr- oder hülsenförmig und so ausgebildet, dass das innenliegende zweite Gehäuseteil 6 in das außenliegende erste Gehäuseteil 4 in Längsrichtung 8 eingeführt werden kann.

Das erfindungsgemäße Handhabungsinstrument 2 kann mit einem Knochenanker lösbar fixiert werden, wobei das außenliegende erste Gehäuseteil 4 einen Kopf des Knochenankers in der Längsrichtung 8 hintergreift und das innenliegende zweite Gehäuseteil 6 gegen den Kopf des Knochenankers in der Längsrichtung 8 abstützbar ist. Das außenliegende erste Gehäuseteil 4 weist hierfür zwei diametral zueinander angeordnete halbschalenförmige Schenkel 18, 20 auf, die derart geringfügig quer zur Längsrichtung 8, d.h. in Richtung der Pfeile 22, 24, auslenkbar sind, dass sie in eine Hintergriffsstellung am Kopf des Knochenankers gelangen.

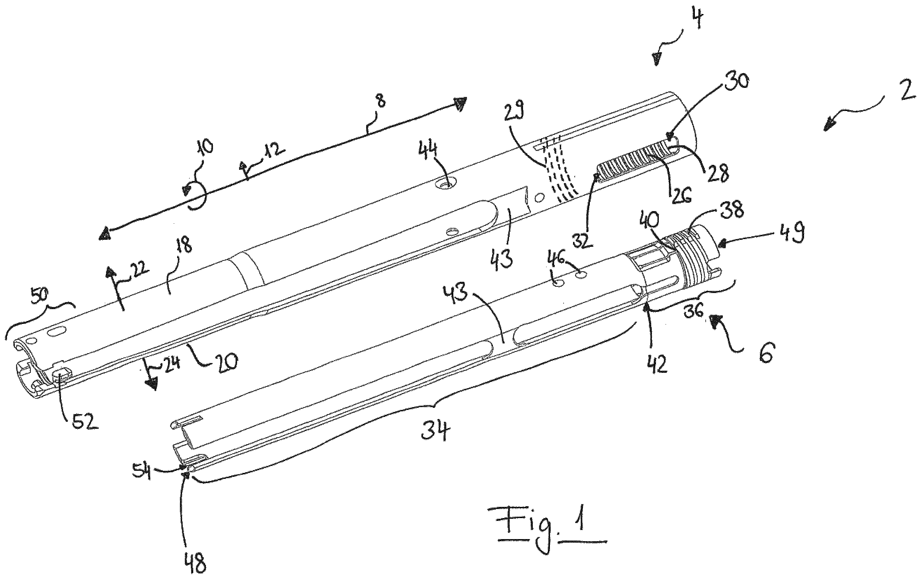

Damit das innenliegende zweite Gehäuseteil 6 in das außenliegende erste Gehäuseteil 4 translatorisch einsteckbar und anschließend einschraubbar ist, umfasst das außenliegende erste Gehäuseteil 4 ein Innengewinde 26 mit einem proximalen Ende 28 und einem nichtersichtlichen, in Fig. 1 angedeuteten, distalen Ende 29. Des Weiteren ist eine fensterartige Ausnehmung im Bereich des Innengewindes 26, die einen gewindelosen Abschnitt 30 mit einem distalen Ende 32 bildet, ersichtlich. Der gewindelose Abschnitt 30 erstreckt sich ausgehend vom proximalen Ende 28 des Innengewindes 26 in der Längsrichtung. Auf der in radialer Richtung 12 gegenüberliegenden Seite des außenliegenden ersten Gehäuseteils 4 ist ebenfalls eine derartige Ausnehmung vorgesehen.

Das innenliegende zweite Gehäuseteil 6 umfasst einen distalen Abschnitt 34, einen proximalen Abschnitt 36 mit Außengewinde 38 und ein Drehgelenk 42 zwischen dem distalen 34 und dem proximalen Abschnitt 36. Wie in Figur 1 ersichtlich, ist das Außengewinde 38, das ein distales Längsende 40 umfasst, nicht über einen gesamten Umfang des innenliegenden zweiten Gehäuseteils 6 in Umfangsrichtung 10 erstreckt, sondern nur in einem zu dem gewindelosen Abschnitt 30 des außenliegenden ersten Gehäuseteils 4 komplementären Bereich vorgesehen. Das Außengewinde 38 erstreckt sich nur über ein Umfangswinkelsegment, das geringfügig kleiner ist, als das Umfangswinkelsegment über das sich der gewindelose Abschnitt 30 erstreckt. Auf der in radialer Richtung 12 gegenüberliegenden Seite des innenliegenden zweiten Gehäuseteils 6 ist ebenfalls ein derartiges Gewinde vorgesehen.

Das innenliegende zweite Gehäuseteil 6 ist bei geeigneter Drehstellung in der Längsrichtung 8 in das außenliegende erste Gehäuseteil 4 translatorisch einsteckbar. Für eine geführte Aufnahme des innenliegenden zweiten Gehäuseteils 6 in das außenliegende erste Gehäuseteil 4, verfügen die Gehäuseteile 4, 6 über Längsführungsmittel 43 in Form von komplementär zueinander ausgebildeten Wandbereichen. Das innenliegende zweite Gehäuseteil 6 ist demnach in das außenliegende erste Gehäuseteil 4 translatorisch einsteckbar, wenn sich die Längsführungsmittel 43 der beiden Gehäuseteile 4, 6 in geeignet fluchtender Stellung zueinander befinden und das Außengewinde 38 des innenliegenden zweiten Gehäuseteils 6 mit dem gewindelosen Abschnitt 30 des außenliegenden zweiten Gehäuseteils 4 fluchtet.

Das innenliegende zweite Gehäuseteil 6 ist so weit in das außenliegende erste Gehäuseteil 4 translatorisch einsteckbar bis das distale Längsende 40 des Außengewindes 38 auf das distale Ende 32 des gewindelosen Abschnitts 30 trifft. Dann ist das Außengewinde 38 in das Innengewinde 26 des außenliegenden ersten Gehäuseteils 4 einschraubbar. Dabei wird nur der proximale Abschnitt 36 des innenliegenden zweiten Gehäuseteils 6 in Umfangsrichtung 10 um die axiale Längsrichtung 8 gedreht, der distale Abschnitt 34 des innenliegenden zweiten Gehäuseteils 6 ist durch das Drehgelenk 42 von der Drehbewegung entkoppelt und wird, geführt durch die Längsführungsmittel 43, weiterhin in der Längsrichtung 8 eingeführt.

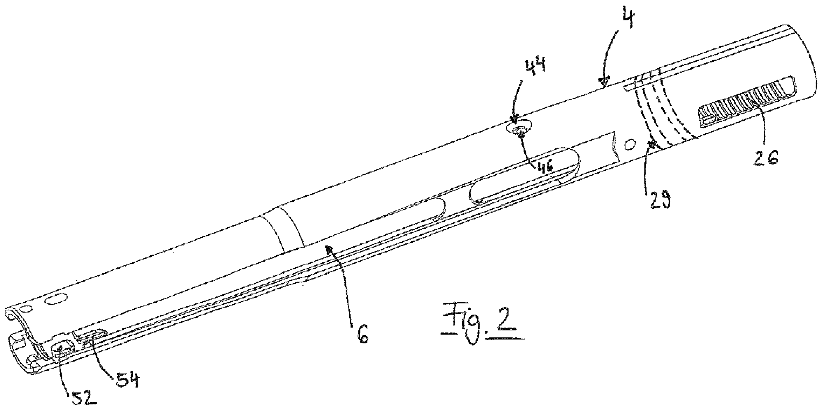

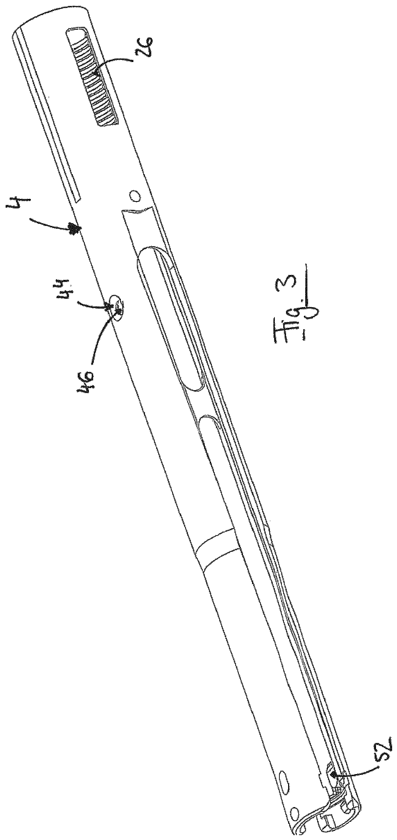

Die Figuren 2 und 3 zeigen das außenliegende erste Gehäuseteil 4 mit eingeführtem innenliegendem zweitem Gehäuseteil 6. Das außenliegende erste Gehäuseteil 4 weist eine radiale Ausnehmung 44 auf, durch die eine visuell wahrnehmbare Markierung 46, welche auf dem innenliegenden zweiten Gehäuseteil 6 angebracht ist, sichtbar ist.

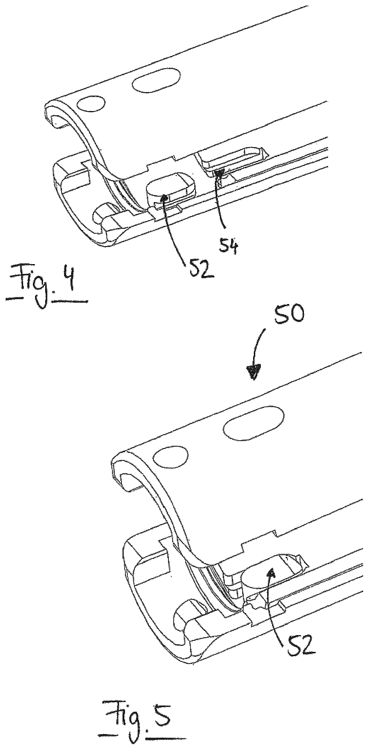

Die Figuren 4 und 5 zeigen als Einzelheit von den Figuren 2 und 3 ein distales Ende 48 des innenliegenden zweiten Gehäuseteils 6 in einem distalen Endbereich 50 des außenliegenden ersten Gehäuseteils 4. Ein radial nach innen vorstehender Vorsprung 52 des außenliegenden ersten Gehäuseteils 4 ist in der Längsrichtung 8 betrachtet beispielhaft T-förmig ausgebildet. In Figur 5 wurde der radial nach innen vorstehende Vorsprung 52 des außenliegenden ersten Gehäuseteils 4 von einer zu dem Vorsprung 52 komplementären Ausnehmung 54 des innenliegenden zweiten Gehäuseteils 6 aufgenommen. Der Vorsprung 52 und die Ausnehmung 54 bilden eine Schwalbenschwanzführung zur formschlüssigen Führung der Gehäuseteile 4, 6. Das innenliegend zweite Gehäuseteil 6 kann soweit in des außenliegend erste Gehäuseteil 4 eingeführt und eingeschraubt werden, bis seine Stirnseite sich an dem Kopf des Knochenankers abstützt.

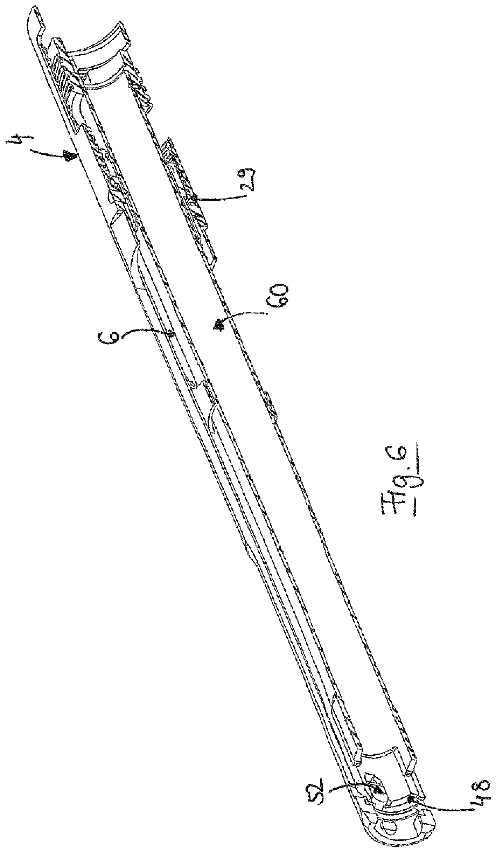

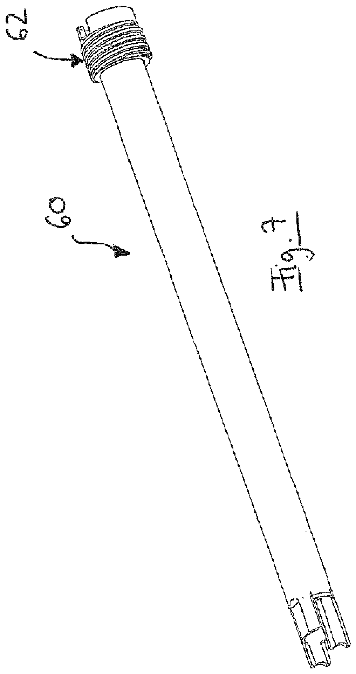

Die Figur 7 zeigt ein insgesamt mit dem Bezugszeichen 60 bezeichnetes Stabdruckteil. Damit das Stabdruckteil 60 in das Innengewinde 26 des außenliegenden ersten Gehäuseteils 4 einschraubbar ist, umfasst das Stabdruckteil 60 ein Außengewinde 62. Es kann dann eine Stellkraft auf einen, in den Knochenanker eingelegten Korrekturstab ausüben. Die Figur 6 zeigt eine Längsschnittansicht des Handhabungsinstruments 2 mit dem darin befindlichen Stabdruckteil 60.

## Patentansprüche

1. Handhabungsinstrument (2) für einen Knochenanker, insbesondere eine Knochenschraube, insbesondere eine Pedikelschraube in der Wirbelsäulenchirurgie, wobei das Instrument mit einem Kopf des Knochenankers lösbar, jedoch starr und drehfest verbindbar ist und in einer axialen Längsrichtung (8) erstreckt ist, mit einem in der Längsrichtung erstreckten außenliegenden ersten Gehäuseteil (4) und mit einem in der Längsrichtung (8) erstreckten in das außenliegende erste Gehäuseteil (4) in der Längsrichtung (8) einführbaren innenliegenden zweiten Gehäuseteil (6), wobei die Gehäuseteile wenigstens abschnittsweise rohr- oder hülsenförmig ausgebildet sind und das außenliegende erste Gehäuseteil (4) ein in der Längsrichtung (8) erstrecktes Innengewinde (2) und das innenliegende zweite Gehäuseteil (6) ein in der Längsrichtung (8) erstrecktes Außengewinde (38) aufweist, das in das Innengewinde (26) einschraubbar ist, wobei das außenliegende erste Gehäuseteil (4) den Kopf des Knochenankers in der Längsrichtung (8) hintergreift und das innenliegende zweite Gehäuseteil (6) im eingeschraubten Zustand gegen den Kopf des Knochenankers in der Längsrichtung (8) abstützbar ist, so dass das Instrument hierdurch am Kopf des Knochenankers lösbar fixierbar ist, **dadurch gekennzeichnet, dass** das außenliegende erste Gehäuseteil (4) im Bereich des Innengewindes (26) einen gewindelosen Abschnitt (30) aufweist, der von einem proximalen Längsende (28) des Innengewindes (26) ausgehend in der Längsrichtung (8) distal erstreckt ist und über ein Winkelsegment in einer Umfangsrichtung (10) erstreckt ist, und dass das Außengewinde (38) des innenliegenden zweiten Gehäuseteils (6) ausgehend von einem distalen Längsende (40) des Außengewindes (38) nur im Bereich der Erstreckung des gewindelosen Abschnitts (30) des ersten außenliegenden Gehäuseteils (4) ausgebildet ist, so dass das zweite innenliegende Gehäuseteil (6) bei geeigneter Drehstellung in der Längsrichtung (8) in das erste außenliegende Gehäuseteil (4) translatorisch einsteckbar ist, bis das distale Längsende (40) des Außengewindes (38) auf ein distales Ende (32) des gewindelosen Abschnitts (30) trifft und im Folgenden in das Innengewinde (26) des ersten außenliegenden Gehäuseteils (4) einschraubbar ist.

2. Handhabungsinstrument (2) nach Anspruch 1, **dadurch gekennzeichnet, dass** der gewindelose Abschnitt (30) des ersten außenliegenden Gehäuseteils (4) dadurch gebildet ist, dass das erste außenliegende Gehäuseteil (4) dort eine radial zur Längsrichtung (8) ausgebildete und nach außen durchgehende Ausnehmung in seiner Wandung aufweist.

3. Handhabungsinstrument (2) nach Anspruch 1 oder 2, **dadurch gekennzeichnet, dass** der gewindelose Abschnitt (30) des ersten außenliegenden Gehäuseteils (4) in Umfangsrichtung (10) eine Erstreckung von 3 bis 20 mm und in der Längsrichtung (8) von 5 bis 100 mm aufweist.

4. Handhabungsinstrument (2) nach einem oder mehreren der vorhergehenden Ansprüche, **dadurch gekennzeichnet, dass** das erste außenliegende Gehäuseteil (4) zwei gewindelose Abschnitte aufweist, die diametral zur Längsrichtung (8) einander gegenüberliegend ausgebildet sind.

5. Handhabungsinstrument (2) nach Anspruch 4, **dadurch gekennzeichnet, dass** das Außengewinde (38) des zweiten innenliegenden Gehäuseteils ausgehend von einem distalen Längsende (40) des Außengewindes (38) nur im Bereich der zwei gewindelosen Abschnitte ausgebildet ist.

6. Handhabungsinstrument (2) nach einem oder mehreren der vorhergehenden Ansprüche, **dadurch gekennzeichnet, dass** das erste außenliegende Gehäuseteil (4) zwei diametral zueinander angeordnete halbschalenförmige Schenkel (18, 20) aufweist, die derart geringfügig quer zur Längsrichtung (8) gegeneinander auslenkbar sind, dass sie in eine Hintergriffsstellung am Kopf des Knochenankers gelangen, insbesondere einrasten oder einschnappen, können und dabei ein proximales Ende des Kopfs des Knochenankers zwischen sich aufnehmen.

7. Handhabungsinstrument (2) nach einem oder mehreren der vorhergehenden Ansprüche, **dadurch gekennzeichnet, dass** das zweite innenliegende Gehäuseteil (6) einen ersten distalen Abschnitt (34) und einen das Außengewinde (38) aufweisenden proximalen Abschnitt (36) aufweist, die über ein Drehgelenk (42) gegeneinander um die axiale Längsrichtung (8) verdrehbar sind.

8. Handhabungsinstrument (2) nach Anspruch 7, **dadurch gekennzeichnet, dass** der erste distale Abschnitt (34) des zweiten innenliegenden Gehäuseteils (6) über Längsführungsmittel (43) unverdrehbar zu dem ersten außenliegenden Gehäuseteil (4) darin geführt aufnehmbar ist.

9. Handhabungsinstrument (2) nach Anspruch 8, **dadurch gekennzeichnet, dass** die Längsführungsmittel (43) die Aufnahme des zweiten innenliegenden Gehäuseteils (6) in dem ersten außenliegenden Gehäuseteil (4) nur in einer diskreten Drehstellung zulassen.

10. Handhabungsinstrument (2) nach einem oder mehreren der vorhergehenden Ansprüche, **dadurch gekennzeichnet, dass** in einem distalen Endbereich (50) des ersten außenliegenden Gehäuseteils (4) wenigstens ein nach radial innen vorstehender Vorsprung (52) ausgebildet ist, der ein distales Längsführungsmittel für das zweite innenliegende Gehäuseteil (6) bildet, wobei das zweite innenliegende Gehäuseteil (6) eine mit dem Vorsprung (52) zusammenwirkende Ausnehmung (54) aufweist.

11. Handhabungsinstrument (2) nach Anspruch 10, **dadurch gekennzeichnet, dass** der Vorsprung (52) in der Längsrichtung (8) oder im Schnitt betrachtet pilzförmig oder T-förmig ausgebildet ist.

12. Handhabungsinstrument (2) nach einem der Ansprüche 10 oder 11, **dadurch gekennzeichnet, dass** die Ausnehmung (54) in dem zweiten innenliegenden Gehäuseteil (6) ausgehend von einem distalen Ende (48) des zweiten innenliegenden Gehäuseteils (6), randoffen und in der Längsrichtung (8) erstreckt ausgebildet ist.

13. Handhabungsinstrument (2) nach einem oder mehreren der vorhergehenden Ansprüche, **dadurch gekennzeichnet, dass** in dem ersten außenliegenden Gehäuseteil (4) wenigstens eine radiale Ausnehmung (44) ausgebildet ist, durch welche hindurch eine Markierung (46) an der Außenseite des zweiten innenliegenden Gehäuseteils (6) visuell wahrnehmbar ist, wenn sich das zweite innenliegende Gehäuseteil (6) in einer bestimmten Position in Längsrichtung (8) relativ zu dem ersten außenliegenden Gehäuseteil (4) befindet.

14. Handhabungsinstrument (2) nach einem oder mehreren der vorhergehenden Ansprüche, **dadurch gekennzeichnet, dass** das innenliegende zweite Gehäuseteil (6) am proximalen Ende (49) eine oder mehrere Werkzeugansetzstellen aufweist und ein passendes Werkzeug so angesetzt werden kann, dass das innenliegende zweite Gehäuseteil (6) über dieses in einer Drehbewegung um die Achse gedreht werden kann.

## Claims

1. Handling instrument (2) for a bone anchor, in particular a bone screw, in particular a pedicle screw in spinal surgery, the instrument being detachably, but also rigidly and non-rotatably, connectable to a head of the bone anchor and extending in an axial longitudinal direction (8), said instrument comprising a first outer housing part (4) extending in the longitudinal direction and a second inner housing part (6) which extends in the longitudinal direction (8) and can be inserted into the first outer housing part (4) in the longitudinal direction (8), the housing parts being tubular or sleeve-shaped, at least in sections, and the first outer housing part (4) including an internal thread (2) which extends in the longitudinal direction (8), and the second inner housing part (6) including an external thread (38) which extends in the longitudinal direction (8) and can be screwed into the internal thread (26), the first outer housing part (4) engaging behind the head of the bone anchor in the longitudinal direction (8), and the second inner housing part (6), when screwed in, being supported against the head of the bone anchor in the longitudinal direction (8) such that the instrument can thus be detachably fastened to the head of the bone anchor, **characterized in that** the first outer housing part (4) includes a threadless section (30) in the region of the internal thread (26), which section extends distally from a proximal longitudinal end (28) of the internal thread (26) in the longitudinal direction (8) and over an angular segment in a circumferential direction (10), and **in that** the external thread (38) of the second inner housing part (6) is formed, from a distal longitudinal end (40) of the external thread (38), only in the region of the extension of the threadless section (30) of the first outer housing part (4) such that the second inner housing part (6) can be plugged into the first outer housing part (4) in the longitudinal direction (8) in a translational manner at a suitable rotational position until the distal longitudinal end (40) of the external thread (38) comes into contact with a distal end (32) of the threadless section (30), and can subsequently be screwed into the internal thread (26) of the first outer housing part (4).

2. Handling instrument (2) according to claim 1, **characterized in that** the threadless section (30) of the first outer housing part (4) is formed by the first outer housing part (4) including a recess in the wall thereof, which recess is radial with respect to the longitudinal direction (8) and continuous towards the outside.

3. Handling instrument (2) according to claim 1 or claim 2, **characterized in that** the threadless section (30) of the first outer housing part (4) includes an extension of from 3 to 20 mm in the circumferential direction (10), and 5 to 100 mm in the longitudinal direction (8).

4. Handling instrument (2) according to one or more of the preceding claims, **characterized in that** the first outer housing part (4) includes two threadless sections which are opposite one another in a manner that is diametrical to the longitudinal direction (8).

5. Handling instrument (2) according to claim 4, **characterized in that** the external thread (38) of the second inner housing part is formed, from a distal longitudinal end (40) of the external thread (38), only in the region of the two threadless sections.

6. Handling instrument (2) according to one or more of the preceding claims, **characterized in that** the first outer housing part (4) includes two mutually diametrical, half-shell-shaped legs (18, 20) which can be slightly deflected, in a manner that is transverse to the longitudinal direction (8), such that they reach a rear-engagement position at the head of the bone anchor, in particular by latching or snapping, and thus receive a proximal end of the head of the bone anchor therebetween.

7. Handling instrument (2) according to one or more of the preceding claims, **characterized in that** the second inner housing part (6) includes a first distal section (34), and a proximal section (36) including the external thread (38), which sections can be rotated relative to one another about the axial longitudinal direction (8) by means of a rotational joint (42).

8. Handling instrument (2) according to claim 7, **characterized in that** the first distal section (34) of the second inner housing part (6) is guided by longitudinal guide means (43) so as to be able to be received in the first outer housing part (4) such that said section cannot rotate relative to said first outer housing part.

9. Handling instrument (2) according to claim 8, **characterized in that** the longitudinal guide means (43) allow the second inner housing part (6) to be received in the first outer housing part (4) only in a discrete rotational position.

10. Handling instrument (2) according to one or more of the preceding claims, **characterized in that** at least one radially inwardly protruding projection (52) is formed in a distal end region (50) of the first outer housing part (4), which projection forms a distal longitudinal guide means for the second inner housing part (6), the second inner housing part (6) including a recess (54) which engages with the projection (52).

11. Handling instrument (2) according to claim 10, **characterized in that** the projection (52) is mushroom-shaped or T-shaped when viewed in the longitudinal direction (8) or in section.

12. Handling instrument (2) according to one of claims 10 or 11, **characterized in that** the recess (54) in the second inner housing part (6), from a distal end (48) of the second inner housing part (6), is open-edged and extends in the longitudinal direction (8).

13. Handling instrument (2) according to one or more of the preceding claims, **characterized in that** at least one radial recess (44) is formed in the first outer housing part (4), through which recess a marking (46) on the outside of the second inner housing part (6) is visually perceptible when the second inner housing part (6) is in a specific position relative to the first outer housing part (4) in the longitudinal direction (8).

14. Handling instrument (2) according to one or more of the preceding claims, **characterized in that** the inner housing part (6) includes, at the proximal end (49), one or more tool application points, and a suitable tool can be applied such that the second inner housing part (6) can be rotated thereby in a rotational movement about the axis.

## Revendications

1. Instrument de manipulation (2) pour un ancre à os, en particulier une vis à os, en particulier une vis pédiculaire en chirurgie rachidienne, dans lequel ledit instrument peut être relié de manière amovible, mais rigide et solidaire en rotation à une tête de l'ancre à os, et s'étend dans une direction longitudinale axiale (8), comprenant une première partie de boîtier (4) située à l'extérieur et s'étendant dans la direction longitudinale ainsi qu'une deuxième partie de boîtier (6) située à l'intérieur et s'étendant dans la direction longitudinale (8) et apte à être insérée dans la première partie de boîtier (4) située à l'extérieur, dans la direction longitudinale (8), dans lequel les parties de boîtier sont réalisées au moins par sections en forme de tube ou en forme de manchon et la première partie de boîtier (4) située à l'extérieur présente un filetage intérieur (2) s'étendant dans la direction longitudinale (8) et la deuxième partie de boîtier (6) située à l'intérieur présente un filetage extérieur (38) qui s'étend dans la direction longitudinale (8) et qui peut être vissé dans le filetage intérieur (26), dans lequel la première partie de boîtier (4) située à l'extérieur se prend derrière la tête de l'ancre à os dans la direction longitudinale (8) et la deuxième partie de boîtier (6) située à l'intérieur peut être appuyée à l'état vissé contre la tête de l'ancre à os dans la direction longitudinale (8) de sorte que l'instrument peut ainsi être fixé de manière amovible à la tête de l'ancre à os, **caractérisé par le fait que** la première partie de boîtier (4) située à l'extérieur présente une section non filetée (30) au niveau du filetage intérieur (26), qui s'étend de manière distale dans la direction longitudinale (8) à partir d'une extrémité longitudinale proximale (28) du filetage intérieur (26) et s'étend sur un segment angulaire dans une direction circonférentielle (10), et que le filetage extérieur (38) de la deuxième partie de boîtier (6) située à l'intérieur n'est réalisé, à partir d'une extrémité longitudinale distale (40) du filetage extérieur (38), qu'au niveau de l'extension de la section non filetée (30) de la première partie de boîtier (4) située à l'extérieur, de sorte que la deuxième partie de boîtier (6) située à l'intérieur peut être insérée en translation, dans une position de rotation appropriée, dans la direction longitudinale (8) dans la première partie de boîtier (4) située à l'extérieur jusqu'à ce que l'extrémité longitudinale distale (40) du filetage extérieur (38) rencontre une extrémité distale (32) de la section non filetée (30) et peut ensuite être vissé dans le filetage intérieur (26) de la première partie de boîtier (4) située à l'extérieur.

2. Instrument de manipulation (2) selon la revendication 1, **caractérisé par le fait que** la section non filetée (30) de la première partie de boîtier (4) située à l'extérieur est formée en ce que la première partie de boîtier (4) située à l'extérieur y présente un évidement dans sa paroi, qui est réalisé radialement à la direction longitudinale (8) et continu vers l'extérieur.

3. Instrument de manipulation (2) selon la revendication 1 ou 2, **caractérisé par le fait que** la section non filetée (30) de la première partie de boîtier (4) située à l'extérieur présente une extension de 3 à 20 mm dans la direction circonférentielle (10) et de 5 à 100 mm dans la direction longitudinale (8).

4. Instrument de manipulation (2) selon l'une ou plusieurs des revendications précédentes, **caractérisé par le fait que** la première partie de boîtier (4) située à l'extérieur présente deux sections non filetées qui sont réalisées de manière à être opposées diamétralement à la direction longitudinale (8).

5. Instrument de manipulation (2) selon la revendication 4, **caractérisé par le fait que** le filetage extérieur (38) de la deuxième partie de boîtier située à l'intérieur n'est réalisé, à partir d'une extrémité longitudinale distale (40) du filetage extérieur (38), qu'au niveau des deux sections non filetées.

6. Instrument de manipulation (2) selon l'une ou plusieurs des revendications précédentes, **caractérisé par le fait que** la première partie de boîtier (4) située à l'extérieur présente deux branches (18, 20) en forme de demi-coque qui sont disposées diamétralement l'une par rapport à l'autre et qui peuvent être déviées légèrement l'une par rapport à l'autre transversalement à la direction longitudinale (8) qu'ils arrivent dans une position dans laquelle elles se prennent derrière la tête de l'ancre à os, en particulier par encliquetage ou enclenchement, tout en recevant ainsi entre elles une extrémité proximale de la tête de l'ancre à os.

7. Instrument de manipulation (2) selon l'une ou plusieurs des revendications précédentes, **caractérisé par le fait que** la deuxième partie de boîtier (6) située à l'intérieur comprend une première section distale (34) et une section proximale (36) présentant le filetage extérieur (38), qui peuvent tourner l'une par rapport à l'autre par l'intermédiaire d'une articulation pivotante (42) autour de la direction longitudinale axiale (8).

8. Instrument de manipulation (2) selon la revendication 7, **caractérisé par le fait que** la première section distale (34) de la deuxième partie de boîtier (6) située à l'intérieur peut être reçue de manière guidée, par l'intermédiaire de moyens de guidage longitudinal (43), dans la première partie de boîtier (4) située à l'extérieur, et de manière à ne pas pouvoir tourner par rapport à celle-ci.

9. Instrument de manipulation (2) selon la revendication 8, **caractérisé par le fait que** les moyens de guidage longitudinal (43) ne permettent la réception de la deuxième partie de boîtier (6) située à l'intérieur dans la première partie de boîtier (4) située à l'extérieur que dans une position de rotation discrète.

10. Instrument de manipulation (2) selon l'une ou plusieurs des revendications précédentes, **caractérisé par le fait que** dans une zone d'extrémité distale (50) de la première partie de boîtier (4) située à l'extérieur est formée au moins une projection (52) qui fait saillie radialement vers l'intérieur et qui forme un moyen de guidage longitudinal distal pour la deuxième partie de boîtier (6) située à l'intérieur, dans lequel ladite deuxième partie de boîtier (6) située à l'intérieur présente un évidement (54) qui coopère avec la projection (52).

11. Instrument de manipulation (2) selon la revendication 10, **caractérisé par le fait que** la projection (52), vue dans la direction longitudinale (8) ou en coupe, est en forme de champignon ou en forme de T.

12. Instrument de manipulation (2) selon l'une quelconque des revendications 10 ou 11, **caractérisé par le fait que** l'évidement (54) dans la deuxième partie de boîtier (6) située à l'intérieur est réalisé, à partir d'une extrémité distale (48) de la deuxième partie de boîtier (6) située à l'intérieur, à bord ouvert et en s'étendant dans la direction longitudinale (8).

13. Instrument de manipulation (2) selon l'une ou plusieurs des revendications précédentes, **caractérisé par le fait que** dans la première partie de boîtier (4) située à l'extérieur est réalisé au moins un évidement radial (44) à travers lequel un marquage (46) se trouvant sur la face extérieure de la deuxième partie de boîtier (6) située à l'intérieur est visuellement perceptible lorsque la deuxième partie de boîtier (6) située à l'intérieur se trouve dans une position déterminée dans la direction longitudinale (8) par rapport à la première partie de boîtier (4) située à l'extérieur.

14. Instrument de manipulation (2) selon l'une ou plusieurs des revendications précédentes, **caractérisé par le fait que** la deuxième partie du boîtier (6) située à l'intérieur présente un ou plusieurs points d'application d'outil à l'extrémité proximale (49) et qu'un outil approprié peut être appliqué de telle manière que la deuxième partie de boîtier (6) située à l'intérieur peut être tournée par celui-ci dans un mouvement de rotation autour de l'axe.
